# NEW EUROPEAN PATENT SPECIFICATION

(11) **EP 2 348 832 B2**
(45) Date of publication and mention of the opposition decision: **25.12.2024**
(45) Mention of the grant of the patent: 16.01.2019
(21) Application number: 09824117.7
(22) Date of filing: 29.10.2009
(51) Int. Cl.: A01N 25/00, A61K 31/70, A61K 31/335, A01N 25/04, A61K 45/06, A61K 31/351, A61K 31/7048, A61K 9/00, A61K 31/365

(54) **PRESERVATIVE SYSTEM FOR EMULSION-BASED THERAPEUTIC TOPICAL FORMULATIONS**
KONSERVIERUNGSSYSTEM FÜR THERAPEUTISCHE TOPISCHE FORMULIERUNGEN AUF EMULSIONSBASIS
SYSTÈME DE CONSERVATION POUR FORMULATIONS TOPIQUES THÉRAPEUTIQUES À BASE D'ÉMULSION

(30) Priority: 29.10.2008 US 109325 P
(43) Date of publication of application: 03.08.2011
(73) Proprietor: Arbor Pharmaceuticals, LLC, Atlanta, GA 30328 (US)
(72) Inventor: SPRING, Nicholas, Jenkintown PA 19046 (US); DELANEY, Edward, J., Princeton NJ 08542 (US)
(74) Representative: D Young & Co LLP
(86) International application number: PCT/US2009/062500
(87) International publication number: WO 2010/051348

(56) References cited:
- EP-A2- 0 045 655
- WO-A1-00/42990
- WO-A1-2004/093886
- WO-A2-2008/067054
- WO-A2-2008/137699
- US-A- 4 199 569
- US-A- 4 389 397
- US-A- 5 929 086
- US-A1- 2003 180 352
- US-A1- 2004 167 084
- US-A1- 2005 143 325
- US-A1- 2005 220 742
- US-A1- 2005 220 742
- US-A1- 2007 020 304
- US-B1- 6 927 210
- RAYMOND C ROWE,PAUL J SHESKEY,MARIAN E QUINN: "Handbook of Pharmaceutical Excipients 5th Edition", 31 December 2006, PHARMACEUTICAL PRESS AND THE AMERICAN PHARMACISTS ASSOCIATION, UK, ISBN: 0 85369 618 7, article "butylparaben, methylparaben, propylparaben", pages: 83-85, 466-469, 629-632, XP002706362
- DANIAL LIMINER: "REMINGTON, The Science and Practice of Pharmacy", 2000, article DANIAL LIMINER: "chapter 44: medicalted topicals", pages: 836 - 857
- AINLEY WADE AND JAMES E. F. REYNOLDS: "Martindale: The Extra Pharmacopoeia. 27th ed.", 1977, THE PHARMACEUTICAL PRESS, article "Preservatives and antioxidants", pages: 1272 - 1287
- CHANDRA MOHAN: "Buffers. A guide for the preparation and use of buffers in biological systems", CALBIOCHEM, 2003
- NAGAOKA ET AL.: "Antimicrobial activity of sodium citrate against Streptococcus pneumoniae and several oral bacteria : Antibacterial activity of citrate", LETTERS IN APPLIED MICROBIOLOGY, vol. 51, no. 5, 1 November 2010 (2010-11-01), pages 546 - 551
- PHILLIPS C A: "The effect of citric acid, lactic acid, sodium citrate and sodium lactate, alone and in combination with nisin, on the growth of Arcobacter butzleri", LETTERS IN APPLIED MICROBIOLOGY, vol. 29, no. 6, 1 December 1999 (1999-12-01), pages 424 - 428
- US provisional application number 61/109,325
- PCT Request Form for WO 2010051348

## Description

### FIELD OF THE INVENTION

The invention relates generally to the field of formulation chemistry. More particularly, the invention relates to Improved preservative systems for emulsion-based therapeutic topical formulations, especially improved preservative systems for emulsion-based ivermectin formulations. The invention relates to topical emulsions in which the aqueous phase is buffered within an acidic pH range within which the preservative system provides a product shelf life significantly longer than the same emulsion-based product without buffer, and, as the therapeutic active is ivermectin, base-catalyzed isomerization is also inhibited.

### BACKGROUND OF THE INVENTION

Emulsion formulations serve as useful vehicles for topical delivery of therapeutic actives. Emulsions, which combine oil and water phases in a single fluid having a monolithic appearance, have several advantages not offered by water-based or oil-based topical therapeutic compositions. First, by combining oil and water phases, emulsions make it possible to formulate hydrophilic and hydrophobic ingredients in a single composition. Second, despite having an oil component, emulsions appeal to consumers because they do not have an oily feel as palpable as purely oil-based formulations.

Third, and perhaps most important to topical therapeutics, emulsions have a rheology wherein they are stable at rest but exhibit a decreasing viscosity as an increasing shear stress is applied. This allows for ease of topical application, but, once applied, the emulsion does not run or drip, and remains in the area where applied that requires therapeutic treatment.

This is particularly advantageous with topical compositions for the treatment of head lice. The product must remain securely where applied for a period of time effective to eradicate the lice. A watery product that quickly runs down the forehead and neck is both unpleasant to apply and ineffective.

Emulsions should remain stable to retain their desirable rheological properties. Separation of the oil and water components will result in a drop in viscosity and produce a drippy, runny product with an oily feel that will not remain in place when applied. Emulsion-based topical therapeutic products should remain stable over their intended useful life.

The therapeutic active must also remain stable. Avermectins are known to undergo base-catalyzed isomerization to form a 2-epimer impurity that has substantially reduced biological activity in comparison to the base product.

Furthermore, many oils and other emulsion components suitable for topical application serve as a nutritional base for microorganisms such as fungi and bacteria. If unimpeded, microbial growth in the formulation may pose a risk of infection to a patient who has been bitten by head lice. In addition, the resulting microbial biomass has an unpleasant odor and appearance, and may otherwise make the product unsuitable for skin contact, There is a need for improvement to the preservative systems of emulsion formulations,

US 2007/0020304 A1 describes an insecticide composition suitable for treating by topical application a subject infested with a parasitic anthropode or to prevent infestation by an arthropod. The insecticide composition is a foamable composition, including an insecticide dissolved in an oil phase, at least on organic carrier such as a hydrophobic organic carrier, a surface-active agent, at least one polymeric agent such as a bioadhesive agent, water and liquefied or compressed gas propellant.

US 2004/167084 A1 is related to formulations for the topical treatment of dermatological conditions comprising as the active ingredient an avermectin compound, such as ivermectin, and a commercial emulsion which is used as carrier for the active ingredient and may comprise as ingredients water, a suspending agent, such as macadamia nut oil, a nonionic surfactant, such as ceteareth-20, and a water soluble preservative, such as phenoxyethanol.

WO 2004/093886 A1 describes a topical pharmaceutical composition intended for human use and the treatment of dermatological conditions. The composition is an emulsion comprising an oily phase comprising fatty substances as suspending agents, surfactant-emulsifiers including nonionic surfactants, ivermectin, preservative agents, such as parabens, and water.

WO 2008/067054 A2 describes a topical formulation for treating head-lice infestation which comprises an avermectin, a suspending agent, surface-active agents, nonionic surfactant, water and preservatives, wherein the formulation can be in the form of emulsions.

### SUMMARY OF THE INVENTION

The above-mentioned problems are solved by the teaching of the independent claims

The invention features topical formulations resistant to the growth of microorganisms, including bacteria, yeast, fungi, molds, and the like. The formulations generally comprise an effective amount of an insecticide dissolved in an oil phase comprising a water-miscible or water-soluble surface active agent, a suspending agent, and a non-ionic surfactant, and an aqueous phase comprising one or more preservatives and which is buffered to a pH at which the formulation is resistant to microorganism growth to a greater extent or is otherwise microbicidal relative to the equivalent formulation in which the aqueous phase is not buffered or buffered differently.

Exemplary insecticides comprise an avermectin such as ivermectin, doramectin, selamectin, or abamectin, or combinations thereof. According to the present invention, ivermectin is used. In exemplary embodiments the insecticide can further comprise a spinosyn, such as spinosyn factors A, B, C, D, E, F, G, H, J, K, L, M, N, 0, P, Q, R, S, T, U, V, W and Y, or combinations thereof. Spinosad is a preferred spinosyn. Ivermectin is included at a concentration of 0.1 to 1% by weight of the formulation. Where ivermectin and spinosad are used, they can be present at a combined concentration of 0.1 % to 5% by weight of the formulation.

According to the invention, the pH is buffered in the range of 4.5 to 6.2. The buffering agent is citric acid and sodium citrate, which may be added to a concentration of 0.01% to 0.1% citric acid and from 1.0% to 1.25% sodium citrate, by weight of the formulation.

The oil phase can comprise 20% to 35% of the suspending agent by weight of the formulation, and the suspending agent comprises olive oil and shea butter. The olive oil can comprise 25% to 28% by weight of the formulation, and the shea butter can comprise 1% to 5% by weight of the formulation.

The oil phase can comprise 15% to 45% of the non-ionic surfactant by weight of the formulation, and the non-ionic surfactant can comprise oleyl alcohol, lanolin alcohol, sorbitan tristearate, or combinations thereof. The oil phase can comprise 10% to 20% of the water-miscible or water-soluble surface active agent by weight of the formulation, and the water-miscible or water soluble surface active agent can comprise polysorbate 80, cetyl acetate, acetylated lanolin alcohol, or combinations thereof.

According to the invention, the formulation comprises as a preservative methylparaben and propylparaben, which can be present in the formulation at a combined concentration of 0.01 to 2% by weight of the formulation.

The formulation can comprise a conditioner such as cyclomethicone. The conditioner can be present at a concentration of 1% to 5% by weight of the formulation.

The invention also features methods for enhancing the resistance of a topical emulsion formulation to microbial growth, including bacterial, fungal, mold, and/or yeast growth. The methods generally comprise buffering the aqueous phase of an emulsion to a pH at which a greater amount of microorganism growth in the formulation is inhibited relative to the equivalent formulation in which the aqueous phase is not buffered or buffered to a more acidic, neutral, or basic pH. Preferred emulsions include those described herein.

According to the invention, the emulsion is buffered to a pH from 4.5 to 6.2. The aqueous phase of the emulsion is buffered using citric acid and sodium citrate. As the methods are employed using a formulation comprising ivermectin, the pH also or alternatively inhibits base-catalyzed isomerization of the ivermectin.

Also featured are methods for treating head lice infestation from a susceptible or treatment-resistant strain of head lice in a subject, such as a human. Generally, the methods comprise topically administering to the subject an effective amount of an emulsion formulation as described herein for a period of time sufficient to treat the head lice infestation. Preferably, the formulation is topically administered to the subject in a single dose. The period of time can range from 1 minute to 60 minutes, although shorter or longer periods of time can be used. If multiple administrations are employed, the interval between each dose is preferably from 5 to 9 days.

### DETAILED DESCRIPTION OF THE INVENTION

Various terms relating to the systems, methods, and other aspects of the invention are used throughout the specification and claims. Such terms are to be given their ordinary meaning in the art unless otherwise indicated.

As used in this specification and the appended claims, the singular forms "a," "an," and "the" include plural referents unless the content clearly dictates otherwise.

It has been observed in accordance with the invention that antimicrobial performance in emulsion formulations is enhanced by buffering the aqueous phase to a pH of 4.5 to 6.2. Accordingly, the invention features topical emulsion formulations, including those comprising preservatives, wherein the aqueous phase is buffered to a pH at which the formulation inhibits a greater amount of microorganism growth relative to the equivalent formulation in which the aqueous phase is not buffered. The invention also features methods for enhancing the microbial resistance and/or antimicrobial properties of topical emulsions.

Topical formulations according to the invention can be aqueous dispersions of an oil phase containing a therapeutically active ingredient. The aqueous dispersions may be prepared as water-in-oil emulsions or oil-in-water emulsions, depending upon the rheological properties desired by the product formulator. How the disclosed ingredients of the invention may be formulated as water-in-oil or oil-in-water emulsions is well understood by one of ordinary skill in the formulation of personal care products such as shampoos and hair conditioners.

The formulations of the invention are particularly well-suited for the topical application of a therapeutically active ingredient that is hydrophobic and poorly solvated by water. Thus, the therapeutically active ingredient preferably is dissolved in suitable agents to improve the stability of the active ingredient in water. Preferably, these agents are easily soluble in water or are water miscible, and include, for example, surfactants, including water-soluble or water-miscible surface active agents. Water-soluble and water-miscible surface active agents include compounds that can dissolve a therapeutically active ingredient and stabilize the active ingredient in water. Highly preferred topical emulsions comprise both an oil phase and an aqueous phase, with the active ingredient dissolved in the oil phase. The oil phase preferably comprises three categories of ingredients: a solubilizer, a non-ionic surfactant, and a suspending agent. Active agents

The active agents are preferably pesticides or insecticides, and more preferably insecticides capable of killing lice.

Topical formulations containing avermectins are useful as agents in the treatment or prevention of an infestation of head lice, where the infested lice may be either a susceptible- or treatment-resistant strain of Pediculus humanus capitis. Treatment-resistant strains include those resistant to one or more pesticides/insecticides, and particularly those ordinarily prescribed or indicated for treating lice infections. Suitable avermectins include ivermectin, doramectin, selamectin, avermectin B₁ₐ, avermectin B_{lb}, selamectin, eprinomectin, and abamectin, with ivermectin being used according to the present invention.

One or more avermectins may be present in the formulations at varying concentrations, for example, from about 0.005% to about 5% by weight of the formulation. For example, a 1% avermectin formulation would include 1 gram (g) avermectin per 100 milliliters (ml) of formulation volume.

According to the present invention, ivermectin is present at a concentration of 0.1% to 1% by weight of the formulation. It has been observed in accordance with the present invention that ivermectin concentrations of 0.25%, 0.5% and 1% promote effective killing of a permethrin-resistant strain of head lice. Ivermectin can be utilized as a mixture of over 80% 22,23-dihydroavermectin Bₗₐ and less than 20% 22,23-dihydro-avermectin B_{ib} and preferably a mixture of at least 90% 22,23-dihydroavermectin Biₐ and less than 10% 22,23-dihydroavermectin B_{lb}. An avermectin such as ivermectin can be dissolved in the formulation at 0.05% to 5% by weight, 0.1% to 2% by weight, or 0.25% to 1% by weight, for example. The topical formulations can comprise combinations of at least one avermectin and at least one spinosyn. The spinosyns can be present at varying concentrations at a weight/volume percentage of, for example, from about 0.005% to about 5% by weight of the formulation. A spinosyn can be dissolved in the formulation at 0.05% to 5% by weight, 0.1% to 5% by weight, 0.1% to 2%, or 0.25% to 1% by weight, for example.

Spinosyns include, but are not limited to, individual spinosyn factors A, B, C, D, E, F, G, H, J, K, L, M, N, 0, P, Q, R, S, T, U, V, W, or Y, and any combinations thereof. Spinosad refers to a combination of the spinosyn factors spinosyn A and spinosyn D, where spinosyn A comprises approximately 85% and spinosyn D comprises approximately 15% of the spinosad.

Combinations of spinosyns and avermectins can comprise 0.01 to 5% of the formulation weight, preferably 0.1 to 5% by weight, preferably 0.25% to 2% by weight, and preferably 0.5% to 3% by weight of the formulation.

It is highly preferred that the insecticide or combination of insecticides is dissolved and remains dissolved in the oil phase of an emulsion formulation. It is preferable that the active agent does not precipitate from solution.

### Solubilizers

The oil phase of the emulsion comprises a water-soluble or water miscible surface-active agent. The water-soluble or water-miscible surface active agent can comprise at least 10% of the formulation weight. In some aspects, this agent comprises 10% to 50% of the formulation weight, and in some aspects, this agent comprises 20% to 50% of the formulation weight. In highly preferred aspects, this agent comprises 10% to 20% of the formulation weight.

Any suitable water-soluble or water-miscible surface active agent can be used. Non-limiting examples include polysorbate 80, cetyl acetate, and acetylated lanolin alcohol, or combinations thereof. Crodalan AWS, available from Croda Chemicals, is a suitable reagent the comprises polysorbate 80, cetyl acetate, and acetylated lanolin alcohol.

Polysorbate 80 can be present in the formulation at 5% to 25% by weight, from 10% to 15% by weight, and preferably 11.25% to 13.5% by weight of the formulation. Cetyl acetate can be present in the formulation at 0.5% to 10% by weight, from 1% to 4% by weight, and preferably 1.50% to 3.75% by weight of the formulation. Acetylated lanolin alcohol can be present in the formulation at 0.10% to 3% by weight, from 0.5% to 1% by weight, and preferably 0.15% to 0.75% by weight of the formulation.

In some aspects, having the water-soluble or water-miscible surface active agent bound to the surface of the therapeutically active ingredient can ensure that the active ingredient is stable in the aqueous environment of the emulsion. In some aspects of the invention, the therapeutically active ingredient may be stabilized by pharmaceutically accepted glycols present in the formulation at a level below 30% by weight, such as, for example below 25%, or below 20% or below 15% or below 10% or below 5%. In some highly preferred aspects, the formulation does not include any glycols, especially propylene or polyethylene glycol.

### Suspending Agents

The oil phase of the emulsion comprises olive oil and shea butter. Olive oil is a triacylglyceride, where three fatty acids are tethered to a glycerol backbone, and shea butter is primarily made of palmitic, stearic, oleic, linoleic, and arachidic fatty acids. Although these fatty acids have been used as "home remedies" for removal of head lice from the scalp, they do not kill head lice. Both olive oil and shea butter are viscous materials that slow the movement of adult lice to better remove them. In some aspects, olive oil is present in the formulation at a level of 20% to 30% by weight of the formulation, and preferably from 25% to 28% by weight (e.g., 27.5% by weight). Shea butter can be present in the formulation at a level of 1% to 5% by weight of the formulation, and preferably 2% by weight. Other known suspending agents which can be utilized in the formulations and related methods include, but are not limited to, coconut oil, palm oil, cottonseed oil, vegetable oil, soybean oil, olive oil, peanut oil, corn oil, sunflower oil, safflower oil, jojoba oil, canola oil, shea butter, cocoa butter, milk fat, amaranth oil, apricot oil, argan oil, avocado oil, babassu oil, ben oil, alga-roba oil, coriander seed oil, false flax oil, grape seed oil, hemp oil, kapok seed oil, meadowfoam seed oil, okra seed oil, perilla seed oil, poppy seed oil, prune kernel oil, pumpkin seed oil, quinoa oil ramtil oil, rice bran oil, camellia oil, thistle oil, wheat germ oil and combinations thereof. Fatty acid glycerides can be used and have known use as skin moisturizers.

### Non-Ionic Surfactants

The oil phase of the emulsion comprises one or more non-ionic surfactants. Non-ionic surfactants include compounds that act at the water-air and water-oil interfaces thereby enhancing wetting ability, emulsion stabilization, foaming, rheology, antistatic, lubricity and surface conditioning properties of the emulsion. In some aspects of the invention, a fatty alcohol or a mixture of fatty alcohols can serve as non-ionic surfactants. Apart from additional stabilization of the active ingredient, the non-ionic surfactants have various purposes in the surface chemistry of the formulation, when the formulation is used in a final product such as a body wash or a shampoo-conditioner.

In addition to their surface-active properties, fatty alcohols are emollients that make the skin smoother and act at the water-air and water-oil interfaces, thereby enhancing wetting ability, emulsion stabilization, foaming, rheology, antistatic, lubricity, and surface conditioning properties of the formulation. Emollients include compounds that soften and smooth skin by preventing the skin from losing moisture. Examples of suitable non-ionic surfactants include, without limitations, oleyl alcohol, lanolin alcohol, sorbitan tristearate, bees wax, erucyl alcohol, ricinolyl alcohol, arachidyl alcohol, capryl alcohol, capric alcohol, behenyl alcohol, lauryl alcohol, myristyl alcohol, cetyl alcohol, stearyl alcohol, isostearyl alcohol, oleyl alcohol, palmitoleyl alcohol, linoleyl alcohol, elaidyl alcohol, elaidolinoleyl alcohol, linolenyl alcohol, elaidolinolenyl alcohol, and combinations thereof.

In some preferred aspects, the formulation includes non-ionic surfactants at a combined concentration of 10% to 35% by weight of the formulation, or preferably 15% to 24% by weight, and more preferably 18-24% by weight. Preferred non-ionic surfactants comprise oleyl alcohol, lanolin alcohol, and sorbitan tristearate. In some aspects, oleyl alcohol can be present in the formulation at 5% to 15% by weight of the formulation, and preferably 10% by weight. Lanolin alcohol can be present in the formulation at 3% to 15% by weight, more preferably from 5% to 10% by weight, and more preferably 8% by weight. Sorbitan tristearate is available commercially as Glycomul^{®} TS (Lonza, Inc.) or SPAN 65 as sold by Merck Schuchardt OHG. Sorbitan tristearate is a low HLB ester based surfactant and has many uses in the food and cosmetic industries. The chemical structure of sorbitan tristearate is defined by a cyclic five member ether, with hydroxyl groups, and three fatty acid side chains. Sorbitan tristearate can be present in the formulation at 0.1% to 3% by weight of the formulation, preferably 0.5% by weight.

### Conditioners

A silicone compound can be added to the formulation, in some aspects, preferably to the oil phase of the emulsion, to serve as a skin or hair conditioner. Conditioning agents can change the texture, feel and appearance of human hair. Conditioning agents other than silicone compounds may also be used. In some aspects, the silicone compound can be selected from volatile silicones, of which cyclomethicone is one. Cyclomethicone can act as a conditioner in formulations to be applied to the hair, such as shampoo-conditioners. It gives a soft, silky feel to hair and evaporates quickly leaving little residue. Cyclomethicone can be included in formulations at 1% to 5% by weight of the formulation, and preferably at about 3% by weight. Examples of conditioners that can be used include, but are not limited to, cyclomethicone, dimethicone, hexamethyldisiloxane, octamethyltrisiloxane, decamethyltetrasiloxane, dodecamethylpentasiloxane, polydimethylsiloxanes, and combinations thereof.

### Preservatives

The formulations according to the invention comprises methylparaben and propylparaben as preservatives to inhibit the growth of microorganisms and/or to protect the formulation from chemical breakdown.

In some aspects, the combined concentration of preservatives in the formulation is 0.05% to 2% by weight of the formulation. Methylparaben can be present In the formulation at 0.01% to 2% by weight of the formulation, and preferably 0.1 to 0.3% by weight, and more preferably 0.20% by weight. Propylparaben can be present in the formulation at 0.01% to 1% by weight, and more preferably 0.01% to 0.5% by weight, more preferably 0.05% to 0.1% by weight, and more preferably 0.1% by weight.

### pH Modifiers and Buffering Agents

The formulation according to the invention has a pH from 4.5 to 6.2, a pH between 4.5 and 6.0 is preferred, a pH between 5.0 and 6.0 is more preferred, and a pH between 5.3 and 5.8 being even more preferred. Highly preferred is the pH of human skin. Buffers capable of buffering the aqueous phase to a pH between 4.5 and 6.2 are commonplace and readily identified by those of ordinary skill in the art.

Combinations of citric acid and sodium citrate are used according to the present invention. pH modifiers can be mixed directly into the formulation in their solid form, or can be added as part of a liquid or solid buffer comprising multiple agents.

In some aspects, citric acid is included in the formulation at 0.01% to 0.1% by weight of the formulation, and preferably 0.055% by weight. In some aspects, these concentrations of citric acid are combined with 1% to 1.25% sodium citrate by weight of the formulation, preferably 1.099% by weight of citric acid. The amount of citric acid and/or sodium citrate can vary according to the desired pH to be achieved with the formulation. In highly preferred aspects, 0.055% by weight citric acid is used in combination with 1.099% or 1.1% by weight sodium citrate. These agents can themselves provide antimicrobial activity independent of the preservatives, and in some aspects may be used in lieu of a separate preservative.

Other exemplary preservatives include sodium benzoate, imidazolidinyl ureas such as the GERMALL family of preservatives, including GERMALL PLUS, polyvalent chelating agents such as EDTA and related compounds in various stages of protonation, and citric acid and sodium salts thereof, either alone, or in combinations of sodium salt and free acid. The other exemplary preservatives can also be present in the formula in the same concentration range as the parabens, e.g., at levels of 0.01% to 2%, or alternatively from 0.01% to 0.5% by weight (e.g., 0.05% by weight).

### Humectants

In some aspects, the formulation can further comprise a humectant. Humectants are hygroscopic materials intended to prevent the formulation from drying out during the course of use and prior to rinsing from the skin, hair or scalp. The humectant may also function as a moisturizer in shampoo and conditioner formulations. Humectants are usually molecules with several hydrophilic groups, such as hydroxyl, amine, carboxylic acid groups and esters thereof that provide the molecule the ability to form hydrogen bonds with water molecules.

In some aspects, other components of the formulation serve a dual role as the humectant, such as many of the non-ionic surfactants, including, but not limited to oleyl alcohol, lanolin alcohol, acetylated lanolin alcohol, and the like. In another embodiment, the humectant is chosen from glycerine, glyceryl triacetate, sorbitol, xylitol, maltitol, polydextrose, quillaia, lactic acid, urea, and the like, and mixtures thereof.

### Water

The oil phase mixture comprising the dissolved therapeutically active ingredient, suspending agents, solubilizer, and non-ionic surfactants, can be dispersed in water, or the water can be dispersed in the oil phase In some aspects, the water is deionized. Water acts as a carrier and may be included as warranted for any respective formulation. In the exemplified formulation, water can be present in the formulation at 30% to 40% by weight of the formulation, and preferably 30% to 33% by weight, and preferably 32%.

The addition of the oil phase comprising the therapeutically active ingredient to the deionized water can result in a colloidal suspension of the active ingredient, where micelles form around the active ingredient and are arranged such that hydrophilic heads of the surfactants are in contact with the solvent water molecules and the hydrophobic tails of the surfactants are in contact with the active ingredient. This formulation is especially suitable for delivering therapeutically active ingredients in body washes and shampoo-conditioners, which, for shampoo-conditioners, gives the products a suitable washout and flow behavior, leaving the hair in good condition.

The inventive formulations invention is exemplified by, but not limited to, a topical emulsion formulation as disclosed in Table 1. This formulation is in the consistency of a shampoo-conditioner or cream and at least comprises an effective amount of a therapeutically active ingredient, as well as a solubilizing agent(s), water, a suspending agent(s), a surfactant(s), silicone compound(s), and a preservative(s), in any combination and/or concentration which may be contemplated by the artisan upon review of this specification

A specific therapeutically active ingredient concentration of 0.50% (w/v) is shown in Table 1. This concentration range is presented to exemplify the invention.

**Table 1**

| Ingredient | % (by weight) |
|---|---|
| Therapeutic Active | 0.50 |
| Deionized Water USP | 31.75 |
| Olive Oil NF | 27.75 |
| Crodalan AWS | 15 |
| Citric Acid USP | 0.055 |
| Sodium Citrate USP | 1.099 |
| Oleyl Alcohol NF | 10 |
| Lanolin Alcohol NF | 8 |
| Cyclomethicone NF | 3 |
| Shea Butter | 2 |
| Sorbitan Tristearate | 0.50 |
| Methylparaben NF | 0.25 |
| Propylparaben NF | 0.10 |

Adjustments may easily be incorporated with components, known equivalent components, combinations of components, and respective concentrations to provide alternative formulations for uses disclosed herein.

Thus, the artisan will be aware that the percentage by weight of any component may be adjusted to compensate for the concentration of the active ingredient, the texture or rheology of the topical formulation and whether it is formulated as a water-in-oil or oil-in-water emulsion (e.g., shampoo, cream, gel) and that components may be added at differing concentrations or may be left out of a formulation or substituted with an equivalent component so as to provide for a topical formulation similar to the exemplified topical formulation described herein

The skilled artisan will appreciate that other beneficial agents can be added into a formulation of the instant invention. Such beneficial agents include, without limitation, vitamins, hair dyes, nutrients, anti-dandruff agents and the like. The artisan can properly select the beneficial agent or the combination thereof such that the at least one beneficial agent would not negate the beneficial aspects of the formulation.

The invention also features methods for enhancing the resistance of topical emulsion formulations, including the formulations described and/or exemplified herein, to microbial growth for enhancing the antimicrobial properties of the formulation. The methods generally comprise buffering aqueous phase of the emulsion to a pH at which the formulation inhibits a greater amount of microorganism growth relative to the equivalent formulation in which the aqueous phase is not been buffered. The formulations exhibit an enhanced resistance to growth of bacteria, yeast, fungi, and molds, among other things.

In general, the pH can be adjusted to a basic, neutral, or acidic level according to the formulation or according to the particular preservative being used. According to the invention, the pH is buffered to an acidic pH from 4.5 to 6.2. The aqueous phase can be buffered to a pH from 5 to 6, or any other pH described herein.

The methods comprise adding an effective amount of citric acid and sodium citrate to the aqueous phase of the emulsion. Such an effective amount can vary, and preferably is sufficient to bring the aqueous phase of the formulation to a pH level that enhances the overall antimicrobial efficacy of the formulation. By way of example but not of limitation, the aqueous phase can comprise from 0.01% to 0.1% citric acid and from 1% to 1.25% sodium citrate by weight of the formulation. These agents can themselves provide antimicrobial activity independent of the preservatives, and in some aspects may be used in lieu of a preservative.

In some aspects, the methods are applied to inhibit base-catalyzed isomerization of the active ingredient. Thus, the formulations can be buffered to a pH sufficient to inhibit base-catalyzed isomerization of the ivermectin. For complex liquids, such as the formulations described and exemplified herein, it was previously unknown whether buffering the pH of the aqueous phase could affect the stability of components in the oil phase, in view of the transient exposure of the oil phase components to the aqueous phase. Because basic pH can induce isomerization of avermectins (Pivnichny, JV et al. (1988) J. Agric. Food Chem. 36:826-8), it is now believed that acidifying the pH of the aqueous phase of an emulsion comprising ivermectin dissolved in the oil phase will prevent or reduce isomerization of the ivermectin. Thus, the aqueous phase is buffered. The pH sufficient to inhibit base-catalyzed isomerization of the active may vary according to the particular active used. In the case of ivermectin, the aqueous phase is buffered to a pH from 4.5 to 6.2, including the pH values described and exemplified herein, and is more preferably buffered to the pH of human skin.

The topical formulations can, for example, be prepared by separately preparing the oil phase and aqueous phase, for example, by mixing together the ingredients for each phase, and then by mixing the oil and aqueous phases together according to any means suitable in the art. Various conditions attendant to mixing the oil and aqueous phases together (*e.g*., temperature, rate of heating and cooling, and the like) can be varied in order to ensure proper dispersion and stability of the resultant emulsion.

Further disclosed herein are methods for prophylaxis or elimination of either susceptible or treatment-resistant head lice. The resistance can be resistance to any head lice treatments currently marketed or otherwise known in the art, such as malathion resistance, lindane resistance, pyrethrum resistance, or permethrin resistance. The methods utilize formulations comprising avermectin with or without a spinosyn in a topical formulation, including any formulations described or exemplified herein.

Generally, the methods comprise topically administering to a subject in need of such treatment an effective amount of the topical emulsion formulation of the invention for a period of time sufficient to treat and preferably eliminate the head lice infestation. It is highly preferred that the formulation be administered to the subject in a single dose, although multiple doses can be used as necessary based on the determination of the subject or a medical practitioner, including two, three, four, or more doses.

The formulation, which can be a shampoo-conditioner, can be used once or twice during a seven day period (e.g., day 1 and between day 5 and day 9), as well as three or four times (with an initial application on day 1 followed for a second, third or fourth application at intervals from 5 days to 9 days). At each dosing, the formulation can be applied and remain on the site of infestation, such as the scalp, for from 1 minute to 60 minutes or from 3 minutes to 30 minutes, then rinsed with warm water. The formulation can also be allowed to remain on the site of infestation, such as the scalp, from 5 minutes to 20 minutes, and from 10 minutes to 15 minutes. 10 minutes is highly preferred. The shampoo-conditioner is preferably formulated to leave the hair in good condition while ridding the scalp of lice.

The methods can be applied to any animal, including companion animals and farm animals. Human beings are most preferred.

Dosing can be varied either by altering the avermectin or spinosyn concentration, as noted above, or by increasing the amount of topical formulation applied to the scalp of the human subject. While an ivermectin and spinosad based formulation is useful to practice the invention, other known avermectins beside ivermectin and other known spinosyns beside spinosad are also contemplated and have utility as the active Ingredient component for the invention.

While dosing ranges may vary, a single application (dosage) to the scalp of an ivermectin/spinosad containing formulation of the invention preferably can range from 1 ml to 200 ml. In some preferred aspects, the dose is from 3 ml to 75 ml, more preferably from 50 ml to 120 ml, and more preferably from 100 ml to 120 ml. The dose amount can vary, for example, according to the amount and/or length of the hair. Thus, for example, longer hair may necessitate a larger dose.

In some aspects, at least 60 ml of the topical formulation is applied to totally saturate the roots and to effectively cover the entire scalp area. The practitioner can vary the ivermectin and spinosad concentrations and/or volume of the topical formulation to manipulate the effective amount of ivermectin and spinosad to be administered to the subject.

An exemplary embodiment disclosed relates to multiple doses of the topical ivermectin (with or without spinosad) based formulations of the invention. Multiple applications can include at least one, two, three or four additional dosages beyond the initial dose, with one or possibly two additional doses being preferred.

It is known in the art that similar treatment regimes are presently utilized to treat not only head lice infestations, but also infestations of pubic lice or body lice. Thus, it will be evident that the avermectin-containing formulations of the invention will also be effective in treating not only head lice, but also infestations of the human body of pubic lice and body lice. The formulations of the invention may be used to treat a pubic lice and/or body lice infestation. The core components of the formulation can be altered to provide for a formulation with the consistency of a cream rinse or lotion that may be applied to the affected areas, left on for a period of time as contemplated for treatment of head lice, and then rinsed off. Multiple dosing may also occur as contemplated herein for treating head lice with a formulation as disclosed herein.

The following examples are provided to describe exemplary aspects of the invention in greater detail.

A formulation comprising ivermectin and/or spinosad as a topical shampoo-conditioner for eliminating treatment resistant lice may be prepared as follows:

### EXAMPLE 1 (not in accordance with the invention)

### Formulation of an Ivermectin Shampoo Conditioner

The ivermectin is weighed and pre-dissolved in a vessel containing a water-miscible surface active agent, hereinafter Phase A, i.e., 15.00% w/v of polysorbate 80. Phase A is heated, with mixing, at a constant temperature of 65 °C until the ivermectin is completely dissolved in the surface active agent. Phase A is then poured into vessel containing Phase B, which consists of suspending agents, preservatives, non-ionic surfactants, humectants, and a conditioner agent.

Phase B consists of 27.25% w/v of olive oil, 2.00% w/v of shea butter, 8.00% w/v of lanolin alcohol, 3.00% w/v of cyclomethicone, 0.50% w/v of sorbitan triesterate, 0.20% w/v of methylparaben and 0.05% w/v of propylparaben. Phases A and B are heated, with mixing, at a constant temperature of 85°C until all ingredients are dissolved and/or melted. Concurrently Phase C, consisting of water buffered with 0.055% w/v citric acid and 1.099: w/v sodium citrate, is heated at a constant temperature of 85°C. With vigorous mixing Phases A and B are slowly added to Phase C. Mixing is continued at or near room temperature until a uniform, homogenous mixture is formed, which is then subsequently packaged.

### EXAMPLE 2

### Formulation of an Ivermectin Emulsion

Ivermectin is weighed and dissolved in the water-miscible surface active agents Crodalan AWS and oleyl alcohol and the resulting solution (Phase A) is heated to 60 - 65°C. Suspending agents, non-ionic surfactants, and a conditioner agent, consisting respectively of 27.25% w/v of olive oil and 2.00% w/v of Shea butter, 8.00% w/v of lanolin alcohol and 0.50% w/v of sorbitan triesterate, and 3.00% w/v of cyclomethicone (Phase B) are combined and heated with mixing to 75-80°C and the dissolution of all ingredients is confirmed. Concurrently Phase C, consisting of water buffered with 0.055% w/v citric acid and 1.099% w/v sodium citrate and methyl and isopropyl parabens, is thoroughly mixed and heated to between 75 - 80 deg C. Phase A is premixed with Phase B, and with vigorous mixing, Phase C is subsequently added. Mixing is continued until a uniform, homogenous blend is formed. The temperature of the blend is brought down to room temperature under continued mixing, after which time the compounded formulation is packaged.

### EXAMPLE 3

### USP Antimicrobial Effectiveness Test

Topical ivermectin emulsions comprising solubilizers, non-ionic surfactants, and suspending agents were prepared and screened for antimicrobial efficacy according to the U.S. Pharmacopoeia (USP) Chapter 31, Section 51 (2008), incorporated by reference herein. Ten emulsions were formulated to contain paraben preservatives with or without citric acid and sodium citrate as shown in Table 2 below. The control formulation did not contain citric acid, or sodium citrate.

**Table 2: Ivermectin formulations**

| | Control | 1 | 6 | 8 | 9 (Control) |
|---|---|---|---|---|---|
| Ingredient | % w/w | % w/w | % w/w | % w/w | % w/w |
| Purified Water USP | 33.00 | 31.85 | 26.85 | 31.75 | 32.85 |
| Ivermectin | .50 | 0.50 | 0.50 | 0.50 | 0.50 |
| Crodalan AWS | 15.00 | 15.00 | 15.00 | 15.00 | 15.00 |
| Oleyl Alcohol NF | 10.00 | 10.00 | 10.00 | 10.00 | 10.00 |
| Lanolin Alcohol NF | 8.00 | 8.00 | 8.00 | 8.00 | 8.00 |
| Olive Oil NF | 27.75 | 27.75 | 27.75 | 27.75 | 27.75 |
| Shea Butter | 2.00 | 2.00 | 2.00 | 2.00 | 2.00 |
| Sorbitan Tristearate | 0.50 | 0.50 | 0.50 | 0.50 | 0.50 |
| Cyclomethicone NF | 3.00 | 3.00 | 3.00 | 3.00 | 3.00 |
| Methylparaben NF | 0.20 | 0.20 | 0.20 | 0.20 | 0.20 |
| Propylparaben NF | 0.05 | 0.05 | 0.05 | 0.05 | 0.05 |
| Propylene Glycol USP | x | | | | |
| Butyl para ben NF | x | | | | 0.05 |
| Citric Acid USP Sodium Citrate USP | x | 0.055 | 0.055 | 0.055 | |
| | x | 1.099 | 1.099 | 1.099 | |
| Na₂EDTA | x | | | | |
| Sodium Benzoate | x | | | | |
| Germali Plus - Powder | x | | | | |
| Glycerin USP | x | | 5.00 | | |
| | | | | | |
| Total | 100.00 | 100.00 | 100.00 | 100.00 | 100.00 |

**Table 2 Continued.**

| | 12 | 14 | 17 | 20 (Control) | 25 | 26 |
|---|---|---|---|---|---|---|
| Ingredient | % w/w | % w/w | % w/w | % w/w | % w/w | % w/w |
| Purified Water | 31.75 | 31.65 | 31.70 | 32.90 | 30.85 | 30.85 |
| Ivermectin | .50 | 0.50 | 0.50 | 0.50 | 0.50 | 0.50 |
| Crodalan AWS | 15.00 | 15.00 | 15.00 | 15.00 | 15.00 | 15.00 |
| Oleyl Alcohol NF | 10.00 | 10.00 | 10.00 | 10.00 | 10.00 | 10.00 |
| Lanolin Alcohol NF | 8.00 | 8.00 | 8.00 | 8.00 | 8.00 | 8.00 |
| Olive Oil NF | 27.75 | 27.75 | 27.75 | 27.75 | 27.75 | 27.75 |
| Shea Butter | 2.00 | 2.00 | 2.00 | 2.00 | 2.00 | 2.00 |
| Sorbitan Tristearate | 0.50 | 0.50 | 0.50 | 0.50 | 0.50 | 0.50 |
| Cyclomethicone NF | 3.00 | 3.00 | 3.00 | 3.00 | 3.00 | 3.00 |
| Methylparaben NF | 0.20 | 0.25 | 0.20 | 0.20 | 0.20 | 0.20 |
| Propylparaben NF | 0.05 | 0.10 | 0.05 | 0.05 | 0.05 | 0.05 |
| Propylene Glycol USP | | | | | | 1.00 |
| Butyl para ben NF | | | | | | |
| Citric Acid USP Sodium Citrate USP | 0.055 | 0.055 | 0.055 | | 0.055 | 0.055 |
| | 1.099 | 1.099 | 1.099 | | 1.099 | 1.099 |
| Na₂EDTA | 0.10 | 0.10 | | | | |
| Sodium Benzoate | | | 0.15 | | | |
| Germali Plus - Powder | | | | 0.10 | | |
| Glycerin USP | | | | | 1.00 | |
| | | | | | | |
| total | 100.00 | 100.00 | 100.00 | 100.00 | 100.00 | 100.00 |

Under the USP, compendial articles are divided into four categories, see Table 3. The criteria of antimicrobial effectiveness for these products are a function of the route of administration.

**Table 3. Compendial Product Categories**

| Category | Product Description |
|---|---|
| 1 | Injections, other parenterals including emulsions, otic products, sterile nasal products, and ophthalmic products made with aqueous bases or vehicles. |
| 2 | Topically used products made with aqueous bases or vehicles, nonsterile nasal products, and emulsions, including those applied to mucous membranes. |
| 3 | Oral products other than antacids, made with aqueous bases or vehicles. |
| 4 | Antacids made with an aqueous base. |

The USP test requires cultures of the following microorganisms: *Candida albicans* (ATCC No. 10231), *Aspergillus niger* (ATCC No. 16404), *Escherichia coli* (ATCC No. 8739), *Pseudomonas aeruginosa* (ATCC No. 9027), and *Staphylococcus aureus* (ATCC No. 6538), and the microorganisms used in the test must not be more than five passages removed from the original ATCC culture.

Each container was inoculated with one of the prepared and standardized organisms, and mixed. The volume of the suspension inoculum used was between 0.5% and 1.0% of the volume of the formulation. The concentration of test microorganisms added to the formulation after inoculation was between 1 × 10⁵ and 1 × 10⁶ cfu per mL of the product.

The inoculated containers were incubated according to the conditions set forth in Table 4. Sample each container at the appropriate intervals specified in Table 5 below. The number of cfu present in each test preparation were determined by the plate count procedure for the applicable intervals. Using calculated concentrations of cfu per mL present at the start of the test, the change in log₁₀ values of the concentration of cfu per mL was calculated for each microorganism at the applicable test intervals, and the changes were expressed in terms of log reductions.

**Table 4. Culture Conditions for Inoculum Preparation**

| Organism | Suitable Medium | Incubation Temperature | Inoculum Incubation Time | Microbial Recovery Incubation Time |
|---|---|---|---|---|
| *Escherichia coli* (ATCC No. 8739) | Soybean-Casein Digest Broth; | 32.5 ± 2.5° | 18 to 24 hours | 3 to 5 days |
| | Soybean-Casein Digest Agar | | | |
| *Pseudomonas aeruginosa* (ATCC No. 9027) | Soybean-Casein Digest Broth; | 32.5 ± 2.5° | 18 to 24 hours | 3 to 5 days |
| | Soybean-Casein Digest Agar | | | |
| *Staphylococcus aureus* (ATCC No. 6538) | Soybean-Casein Digest Broth; | 32.5 ± 2.5° | 18 to 24 hours | 3 to 5 days |
| | Soybean-Casein Digest Agar | | | |
| *Candida albicans* (ATCC No. 10231) | Sabouraud Dextrose Agar; | 22.5 ± 2.5° | 44 to 52 hours | 3 to 5 days |
| | Sabouraud Dextrose Broth | | | |
| *Aspergillus niger* (ATCC No. 16404) | Sabouraud Dextrose Agar; | 22.5 ± 2.5° | 6 to 10 days | 3 to 7 days |
| | Sabouraud Dextrose Broth | | | |

The requirements for antimicrobial effectiveness are met if the criteria specified under Table 5 are met. No increase is defined as not more than 0.5 log10 units higher than the previous value measured.

**Table 5. Criteria for Tested Microorganisms**

| For Category 2 Products | |
|---|---|
| Bacteria: | Not less than 2.0 log reduction from the initial count at 14 days, and no increase from the 14 days' count at 28 days. |
| Yeast and Molds: | No increase from the initial calculated count at 14 and 28 days. |

The results of the USP antimicrobial efficacy testing of select formulations from Table 2 is provided in Table 6 below. The "Control" formulation (referenced in Table 2) was determined to be inadequate for inoculation as this formulation had visible growth of mold when stored over a period of months under controlled laboratory conditions, even without the specific inoculations (data not shown). Minor variants of the control formulation subsequently were found to routinely fail the USP antimicrobial efficacy test for *Candida albicans.* (Table 6: Ec = *Escherichia coli*; Pa = *Pseudomonas aeruginosa;* Sa = *Staphylococcus aureus;* Ca = *Candida albicans*; An = *Aspergillus niger*).

**Table 6. Antimicrobial efficacy.**

| | | **7 day** | **14 Day** | **28 Day** |
|---|---|---|---|---|
| **Formulation No.** | **Organism/Dil** | **CFU/g** | **CFU/g** | **CFU/n** |
| | | | | |
| 8 | Ec/-1 | | <10 | <10 |
| | Pa/-1 | | <10 | <10 |
| | Sa/-1 | | <10 | <10 |
| | Ca/-1 | | 235 | <10 |
| | An/-4 | | 6.5 X 10⁵ | 3.0 X 10³ |
| | | | | |
| 17 | Ec/-1 | <10 | <10 | <10 |
| | Pa/-1 | <10 | <10 | <10 |
| | Sa/-1 | <10 | <10 | <10 |
| | Ca/-1 | <10 | <10 | <10 |
| | An/-4 | 1.6 X 10⁵ | 1.2 X 10³ | 30 |
| | | | | |
| 9 | Ec/-4 | 2.6 X 10⁵ | 4.5 X 10³ | TNTC, TNTC |
| | Pa/-1 | 475 | 3.9 X 10³ | TNTC, TNTC |
| | Sa/-4 | 1.2 X 10⁶ | 3.5 X 10⁴ | 2.6 X 10⁴ |
| | Ca/-4 | 8.4 X 10⁵ | 45 | TNTC, TNTC |
| | An/ -4 | 4.4 X 10⁵ | 2.3 X 10⁵ | 9.5 X 10⁴ |
| | | | | |
| 20 | Ec/-1 | <10 | <10 | <10 |
| | Pa/-1 | <10 | <10 | <10 |
| | Sa/-1 | <10 | <10 | <10 |
| | **Ca/-1** | **80** | **<10** | **150** |
| | **An/-1** | **<10** | **<10** | **<10** |
| | | | | |
| **25** | **Ec/-1** | **<10** | **<10** | **<10** |
| | **Pa/-1** | **<10** | **<10** | **<10** |
| | **Sa/-1** | **<10** | **<10** | **<10** |
| | **Ca/-1** | **285** | **<10** | **<10** |
| | **An/-4** | **6.1 X 10³** | **3.8 X 10⁴** | **1.5 X 10⁴** |
| | | | | |
| **26** | **Ec/-1** | **<10** | **<10** | **<10** |
| | **Pa/-1** | **<10** | **<10** | **<10** |
| | **Sa/-1** | **<10** | **<10** | **<10** |
| | **Ca/-1** | **<10** | **<10** | **<10** |
| | **An/-4** | **5.8 X 10³** | **1.6 X 10⁴** | **9.5 X 10⁵** |
| | | | | |
| **6** | **Ec/-1** | **<10** | **<10** | **<10** |
| | **Pa/-1** | **<10** | **<10** | **<10** |
| | **Sa/-1** | **<10** | **<10** | **<10** |
| | **Ca/-1** | **885** | **<10** | **<10** |
| | **An/-4** | **9.0 X 10³** | **1.5 X 10⁵** | **2.4 X 10⁴** |
| | | | | |
| **12** | **Ec/-1** | **<10** | **<10** | **<10** |
| | **Pa/-1** | **<10** | **<10** | **<10** |
| | **Sa/-1** | **<10** | **<10** | **<10** |
| | **Ca/-1** | **465** | **<10** | **<10** |
| | **An/-4** | **3.8 X 10⁵** | **8.5 X 10³** | **9.5 X 10³** |
| | | | | |
| **14** | **Ec/-1** | **<10** | **<10** | **<10** |
| | **Pa/-1** | **<10** | **<10** | **<10** |
| | **Sa/-1** | **<10** | **<10** | **<10** |
| | **Ca/-1** | **475** | **<10** | **<10** |
| | **An/-4** | **4.6 X 10⁵** | **5.0 X 10³** | **8.0 X 10³** |
| | | | | |
| **1** | **Ec/-1** | **3.5 X 10⁵** | **<10** | **<10** |
| | **Pa/-1** | **7.3 X 10⁵** | **<10** | **<10** |
| | **Sa/-1** | **5.5 X 10⁵** | **<10** | **<10** |
| | **Ca/-1** | **1.3 X 10⁵** | **<10** | **<10** |
| | **An/-4** | **5.0 X 10⁵** | **1.2 X 10³** | **3.0 X 10³** |
| (Formulations 9 and 20 above are "control" formulations (referenced in Table 2)) TNTC: Too numerous to count. | | | | |

These data show that the overall antimicrobial efficacy in an emulsion formulation can be enhanced by buffering the dispersed aqueous phase of the emulsion to a pH at which the preservative system inhibits growth for a wider variety of microorganism types in the formulation relative to the equivalent formulation in which the aqueous phase is not buffered.

## Claims

1. A topical formulation that inhibits the growth of microorganisms, comprising ivermectin at a concentration of 0.1 to 1% by weight of the formulation dissolved in an oil phase comprising a water-miscible or water-soluble surface active agent, olive oil and shea butter, and a non-ionic surfactant, and an aqueous phase comprising methylparaben and propylparaben, wherein the aqueous phase is buffered to a pH at which the formulation inhibits a greater amount of microorganism growth relative to the equivalent formulation in which the aqueous phase is not buffered, wherein the aqueous phase is buffered with citric acid and sodium citrate, and wherein the pH is from 4.5 to 6.2.

2. The topical formulation of claim 1, wherein olive oil is present in the formulation at a level of 20% to 30% by weight of the formulation.

3. The topical formulation of claim 1, wherein shea butter is present in the formulation at a level of 1% to 5% by weight of the formulation.

4. The topical formulation of claim 1, wherein the formulation comprises methylparaben and propylparaben at a combined concentration of 0.01% to 2% by weight of the formulation.

5. A method for enhancing the resistance of an emulsion formulation to microbial growth, comprising buffering the aqueous phase of the emulsion with citric acid and sodium citrate to a pH from 4.5 to 6.2, wherein said formulation comprises ivermectin at a concentration of 0.1 to 1% by weight of the formulation dissolved in an oil phase comprising a water-miscible or water-soluble surface active agent, olive oil and shea butter, and a non-ionic surfactant, and an aqueous phase comprising methylparaben and propylparaben, wherein the buffering of the aqueous phase of the emulsion is to a pH at which the formulation inhibits a greater amount of microorganism growth relative to the equivalent formulation in which the aqueous phase is not buffered.

6. A formulation of any one of claims 1 to 4 for use in the treatment of head lice infestations in human or animal subjects in need of such treatment.

7. The formulation of claim 6, wherein the formulation is administered topically to the subject for a period of time sufficient to treat the head lice infestation.

8. The formulation of claim 7, wherein the formulation is topically administered to the subject in a single dose.

9. The formulation of claim 7, wherein the period of time is from 1 minute to 60 minutes.

10. The formulation of claim 7, wherein the period of time is from 3 minutes to 30 minutes.

11. The formulation of claim 7, wherein the period of time is from 5 minutes to 20 minutes.

12. The formulation of claim 7, wherein the period of time is from 10 minutes to 15 minutes.

13. The formulation of claim 7, wherein the period of time is 10 minutes.

## Patentansprüche

1. Topische Formulierung, die das Wachstum von Mikroorganismen hemmt, umfassend Ivermectin in einer Konzentration von 0,1 bis 1 Gew.-% der Formulierung, gelöst in einer Ölphase, die einen wassermischbaren oder wasserlöslichen oberflächenaktiven Stoff, Olivenöl und Sheabutter und ein nichtionisches Tensid umfasst, und einer wässrigen Phase, die Methylparaben und Propylparaben umfasst, wobei die wässrige Phase auf einen pH-Wert gepuffert ist, bei dem die Formulierung eine größere Menge an Mikroorganismenwachstum relativ zu der äquivalenten Formulierung, in der die wässrige Phase nicht gepuffert ist, inhibiert, wobei die wässrige Phase mit Zitronensäure und Natriumcitrat gepuffert ist und wobei der pH-Wert von 4,5 bis 6,2 beträgt.

2. Topische Formulierung nach Anspruch 1, wobei Olivenöl in der Formulierung in einem Anteil von 20 bis 30 Gew.-% der Formulierung vorhanden ist.

3. Topische Formulierung nach Anspruch 1, wobei Sheabutter in der Formulierung in einem Anteil von 1 bis 5 Gew.-% der Formulierung vorhanden ist.

4. Topische Formulierung nach Anspruch 1, wobei die Formulierung Methylparaben und Propylparaben in einer kombinierten Konzentration von 0,01 bis 2 Gew.-% der Formulierung umfasst.

5. Verfahren zur Erhöhung der Beständigkeit einer Emulsionsformulierung gegen mikrobielles Wachstum, umfassend das Puffern der wässrigen Phase der Emulsion mit Zitronensäure und Natriumcitrat auf einen pH-Wert von 4,5 bis 6,2, wobei die Formulierung Ivermectin in einer Konzentration von 0,1 bis 1 Gew.-% der Formulierung, gelöst in einer Ölphase, die einen wassermischbaren oder wasserlöslichen oberflächenaktiven Stoff, Olivenöl und Sheabutter und ein nichtionisches Tensid umfasst, und einer wässrigen Phase, die Methylparaben und Propylparaben umfasst, umfasst, wobei das Puffern der wässrigen Phase der Emulsion bis zu einem pH-Wert erfolgt, bei dem die Formulierung eine größere Menge an Mikroorganismenwachstum relativ zu der äquivalenten Formulierung, in der die wässrige Phase nicht gepuffert ist, inhibiert.

6. Formulierung nach einem der Ansprüche 1 bis 4 zur Verwendung bei der Behandlung von Kopflausbefall bei menschlichen oder tierischen Subjekten, die einer solchen Behandlung bedürfen.

7. Formulierung nach Anspruch 6, wobei die Formulierung dem Subjekt für eine ausreichende Zeitspanne topisch verabreicht wird, um den Kopflausbefall zu behandeln.

8. Formulierung nach Anspruch 7, wobei die Formulierung dem Subjekt in einer Einzeldosis topisch verabreicht wird.

9. Formulierung nach Anspruch 7, wobei die Zeitspanne 1 Minute bis 60 Minuten beträgt.

10. Formulierung nach Anspruch 7, wobei die Zeitspanne 3 Minuten bis 30 Minuten beträgt.

11. Formulierung nach Anspruch 7, wobei die Zeitspanne 5 Minuten bis 20 Minuten beträgt.

12. Formulierung nach Anspruch 7, wobei die Zeitspanne 10 Minuten bis 15 Minuten beträgt.

13. Formulierung nach Anspruch 7, wobei die Zeitspanne 10 Minuten beträgt.

## Revendications

1. Formulation topique qui inhibe la croissance de micro-organismes, comprenant de l'ivermectine à une concentration de 0,1 à 1 % en poids de la formulation dissoute dans une phase huileuse comprenant un agent actif en surface miscible à l'eau ou soluble dans l'eau, de l'huile d'olive et du beurre de karité, et un tensioactif non-ionique, et une phase aqueuse comprenant du méthylparabène et du propylparabène, la phase aqueuse étant tamponnée jusqu'à un pH auquel la formulation inhibe une plus grande quantité de croissance de micro-organismes par rapport à la formulation équivalente dans laquelle la phase aqueuse n'est pas tamponnée, la phase aqueuse étant tamponnée avec de l'acide citrique et du citrate de sodium, et le pH étant de 4,5 à 6,2.

2. Formulation topique selon la revendication 1, dans laquelle l'huile d'olive est présente dans la formulation à un taux de 20 % à 30 % en poids de la formulation.

3. Formulation topique selon la revendication 1, dans laquelle le beurre de karité est présent dans la formulation à un taux de 1 % à 5 % en poids de la formulation.

4. Formulation topique selon la revendication 1, dans laquelle la formulation comprend du méthylparabène et du propylparabène à une concentration combinée de 0,01 % à 2 % en poids de la formulation.

5. Procédé pour augmenter la résistance d'une formulation d'émulsion à la croissance microbienne, comprenant le fait de tamponner la phase aqueuse de l'émulsion avec de l'acide citrique et du citrate de sodium jusqu'à un pH de 4,5 à 6,2, ladite formulation comprenant 0,1 à 1 % en poids de formulation dissoute dans une phase huileuse comprenant un agent actif en surface miscible à l'eau ou soluble dans l'eau, de l'huile d'olive et du beurre de karité, et un tensioactif non-ionique, et une phase aqueuse comprenant du méthylparabène et du propylparabène, le tamponnage de la phase aqueuse de l'émulsion étant jusqu'à un pH auquel la formulation inhibe une plus grande quantité de croissance de micro-organismes par rapport à la formulation équivalente dans laquelle la phase aqueuse n'est pas tamponnée.

6. Formulation selon l'une quelconque des revendications 1 à 4, pour une utilisation dans le traitement d'infestations de poux de tête chez des sujets humains ou animaux nécessitant un tel traitement.

7. Formulation selon la revendication 6, dans laquelle la formulation est administrée par voie topique au sujet pendant une période de temps suffisante pour traiter l'infestation de poux de tête.

8. Formulation selon la revendication 7, dans laquelle la formulation est administrée par voie topique au sujet en une seule dose.

9. Formulation selon la revendication 7, dans laquelle la période de temps est de 1 minute à 60 minutes.

10. Formulation selon la revendication 7, dans laquelle la période de temps est de 3 minutes à 30 minutes.

11. Formulation selon la revendication 7, dans laquelle la période de temps est de 5 minutes à 20 minutes.

12. Formulation selon la revendication 7, dans laquelle la période de temps est de 10 minutes à 15 minutes.

13. Formulation selon la revendication 7, dans laquelle la période de temps est de 10 minutes.
